# EUROPEAN PATENT SPECIFICATION

(11) **EP 3 650 053 B1**
(45) Date of publication and mention of the grant of the patent: **15.01.2025**
(21) Application number: 18829135.5
(22) Date of filing: 06.07.2018
(51) Int. Cl.: A61L 31/02, A61L 31/14, A61L 31/16, A61L 31/08

(54) **IMPLANTABLE APPARATUS**
IMPLANTIERBARE VORRICHTUNG
APPAREIL IMPLANTABLE

(30) Priority: 06.07.2017 CN 201710548407
(43) Date of publication of application: 13.05.2020
(73) Proprietor: Biotyx Medical (Shenzhen) Co., Ltd., Shenzhen, Guangdong 518000 (CN)
(72) Inventor: ZHANG, Deyuan, Shenzhen Guangdong 518057 (CN); LIN, Wenjiao, Shenzhen Guangdong 518057 (CN); QIN, Li, Shenzhen Guangdong 518057 (CN); CHEN, Liping, Shenzhen Guangdong 518057 (CN); SUN, Hongtao, Shenzhen Guangdong 518057 (CN)
(74) Representative: Prinz & Partner mbB
(86) International application number: PCT/CN2018/094861
(87) International publication number: WO 2019/007423

(56) References cited:
- WO-A1-2016/152908
- CN-A- 104 689 378
- CN-A- 106 474 545
- CN-A- 106 606 800
- CN-A- 106 806 938
- US-A1- 2010 076 556
- US-A1- 2012 177 910

## Description

The present invention relates to the field of medical devices, and in particular to an implantable device.

### Background

Currently, in the field of cardiovascular implantation application, vascular stents are generally made of non-degradable metals. The disadvantage of the non-degradable metals is that they cannot be degraded or removed, and it is easy to cause many late-stage adverse events such as late thrombosis and the like. An implantable medical device made of absorbable materials has a good application potential. Absorbable materials commonly used clinically mainly include degradable polymers and corrodible metallic materials. Commonly used corrodible metallic materials include iron, magnesium, zinc and the like.

An implantable device is implanted into a patient after successful percutaneous transluminal angioplasty or other interventional procedures, and the treated blood vessels tend to "heal" quickly after local injury. In the process of "healing", hyperplasia of smooth muscle cells around the device has been shown to become severe, and it is easy to cause narrowing of the local vascular lumen again.

After being implanted into a human body, the corrodible metallic material may generate corrosion products containing a metal ion during corrosion. Studies have shown that a zinc-containing device can inhibit hyperplasia of smooth muscle cells when the zinc released during corrosion in the implanted animal body reaches a certain concentration, and it is inferred that the zinc-containing device can reduce the lumen stenosis rate of the tissues surrounding the device. However, there is also evidence showing that the release of excess zinc ions from the implanted zinc-containing device will kill smooth muscle cells and even endothelial cells and other normal tissue cells, which ultimately leads to ulceration or necrosis of the implant site. The prior art does not address how to inhibit hyperplasia of smooth muscle cells and reduce the stenosis rate, and also to avoid death of smooth muscle cells, endothelial cells and other normal cells caused by the fact that the accumulated concentration of zinc which released from the zinc-containing device in tissues exceeds the toxic concentration after the zinc-containing device is implanted in a lumen of a mammal for a certain time.

Therefore, it is necessary to provide a zinc-containing implantable device which, when being implanted in vivo, can effectively inhibit hyperplasia of smooth muscle cells surrounding the implantable device, reduce the likelihood of stenosis, and also prevent the concentration of zinc corrosion products generated after implantation from accumulating to a high toxic concentration in the surrounding tissues, which would result in death of smooth muscle cells, endothelial cells, and other normal cells.

US 2010/0076556 A1 discloses a biodegradable medical implant comprising 5 to 100% by weight of an metallic material and 95 to 5% by weight of a polymeric material. The metallic material has a crystalline microstructure with an average grain size of between 2 nm and 10 µm and the thickness of the implant ranges from 5 µm to 2.5 cm.

WO 2016/152908 A1 discloses a medical implant comprising a zinc-based alloy produced by extrusion of a sintered material containing zinc and magnesium. The alloy preferably comprises 97 wt.% or more zinc and the average crystal grain size is 5 µm of less. When the implant is a stent, the thickness of the zinc alloy is preferably 50 to 200 µm.

CN 106 606 800 A discloses a method of manufacturing a Zn-Fe alloy for use in preparing a degradable medical implant. The alloy contains up to 90 wt.% zinc and 0 to 10 wt% iron. A surface of the Zn-Fe alloy can be coated with a degradable polymer coating, a ceramic coating or a drug coating.

### Summary

An object of the present invention is to provide an implantable device having a zinc-containing portion that maintains the concentration of zinc corrosion products in the surrounding tissues of the zinc-containing portion of the implantable device within a reasonable range during corrosion of the implantable device in vivo, thereby effectively inhibiting hyperplasia of smooth muscle cells surrounding the implantable device. Meanwhile, the peak concentration of zinc corrosion products in the surrounding tissues of the zinc-containing portion of the implantable device should not reach the toxic concentration that would kill smooth muscle cells, endothelial cells and other normal tissue cells, thereby avoiding cell death and tissue necrosis.

When an implantable device having a zinc-containing portion is implanted into a body, the zinc-containing portion comes into contact with body fluid, then corrodes and generate zinc corrosion products in a variety of forms, including zinc ions, solid compounds of zinc, or complexes of zinc, etc. During corrosion of the zinc-containing portion, the generated zinc corrosion products will be absorbed and metabolized by body tissues, therefore, the zinc corrosion products can be accumulated in the tissues only when the mass of the generated zinc corrosion products per unit time is greater than the mass of the zinc corrosion products metabolized by body tissues. Finally, when the concentration of zinc corrosion products accumulated in the tissues surrounding the zinc-containing portion of the implantable device is higher than the lower limit of the concentration for inhibiting hyperplasia of smooth muscle cells in the organism, the zinc corrosion products can inhibit cell hyperplasia of the surrounding tissues of the zinc-containing portion. Meanwhile, in order to avoid death of smooth muscle cells, endothelial cells or other normal tissue cells in the tissues surrounding the zinc-containing portion, and to prevent tissue ulceration or necrosis, it is also necessary to control the concentration of zinc corrosion products accumulated in the tissues always be lower than the toxic concentration leading to cell death.

Since the mass of zinc corrosion products metabolized by the tissues per unit time is substantially constant, the concentration of zinc corrosion products accumulated in the surrounding tissues correlates with the mass of zinc corrosion products generated per unit time. And the mass of zinc corrosion products produced per unit time is related to both the zinc content of the zinc-containing portion and the corrosion rate of the zinc-containing portion.

With the same corrosion rate of zinc, the higher the zinc content of the zinc-containing portion, the larger the mass of zinc in contact with body fluid per unit time, and therefore the larger the mass of zinc corrosion products generated per unit time; the lower the zinc content in the zinc-containing portion, the less the mass of zinc in contact with body fluid per unit time, and therefore the mass of zinc corrosion products generated per unit time.

With the same zinc content in the zinc-containing portion, when the zinc corrosion rate of the zinc-containing portion is faster, the mass of zinc corrosion products generated per unit time is larger. And the corrosion rate of zinc is closely related to the microstructure of zinc. For example, when the microstructure of zinc is an amorphous structure, the corrosion rate of zinc is faster; when the microstructure of zinc is a non-equiaxed structure, an ultrafine-grained structure or an equiaxed structure with a micro-grain size number of 7 to 14, the corrosion rate of zinc is slower. Moreover, the corrosion rate of the non-equiaxed structure and the equiaxed structure with a micro-grain size number of 7 to 14 is slower than that of the ultrafine-grained structure.

The mass of zinc corrosion products generated per unit time should match the zinc content in the zinc-containing portion with the microstructure of zinc, therefore the concentration of the zinc corrosion products accumulated in the tissues surrounding the zinc-containing portion of the device can be controlled to inhibit hyperplasia of smooth muscle cells, while ensuring that the peak concentration of zinc corrosion products does not cause death of smooth muscle cells, endothelial cells or other normal tissue.

According to the present invention, an implantable device having at least one corrodible zinc-containing portion is provided as defined in claim 1. The zinc content in the at least one zinc-containing portion ranges from (70, 100] wt.%, and the microstructure of zinc in the zinc-containing portion is at least one of a non-equiaxed structure, an ultrafine-grained structure, or an equiaxed structure having a micro-grain size number of 7 to 14.

When the implantable device is implanted in vivo, the zinc-containing portion is corroded in contact with body fluid to generate zinc corrosion products. When the concentration of the zinc corrosion product accumulated in the tissues surrounding the zinc-containing portion is higher than the concentration inhibiting hyperplasia of smooth muscle cells, cell hyperplasia of the surrounding tissues can be inhibited, and meanwhile the concentration of the zinc corrosion product in the surrounding tissues may be always lower than the toxic concentration causing death of smooth muscle cells, endothelial cells or other normal tissue cells. Therefore, the implantable device can effectively inhibit hyperplasia of the tissues surrounding the zinc-containing portion, and also can prevent the tissues surrounding the zinc-containing portion from ulceration or necrosis.

When the implantable device is a vascular stent, the luminal stenosis rate is taken as the indicator concerning the effect of inhibiting hyperplasia by zinc corrosion products. The luminal stenosis rate can be obtained from surveying the pathological section of the tissues surrounding the zinc-containing portion and calculating, and the calculation formula is expressed as: Luminal Stenosis rate = (Original lumen area-Existing lumen area)/Original lumen area × 100%.

As can be seen, the lumen stenosis rate reflects the narrowing degree of a lumen. A lower luminal stenosis rate indicates a better effect of inhibiting hyperplasia and higher luminal stenosis rate indicates a poor effect of inhibiting hyperplasia. Embodiments of the present disclosure defines that when the luminal stenosis rate is greater than or equal to 75%, stenosis occurs in the lumen.

It can be understood that the greater the mass of zinc corrosion product generated per unit time, the shorter the period of time needed to achieve an effective concentration for inhibiting hyperplasia of smooth muscle cells in the tissues surrounding the zinc-containing portion, thus the earlier the effect of inhibiting hyperplasia of smooth muscle cells can be achieved, and the luminal stenosis rate after implantation of the device can be further reduced. When the implantable device is implanted into the lumen, the luminal tissue will be injured, and during the critical period of luminal tissue repair, hyperplasia of smooth muscle cells is the most severe, which will slow down and finally reach stable after passing through the critical period of luminal tissue repair. Therefore, the luminal stenosis rate increases most clearly during the critical period of lumen repair, and it will increase very slowly in the short term and tend to be stable in the long term once passing through the critical period of lumen repair. As for vascular stents, the critical period of vascular repair is one month. Therefore, smooth muscle cells proliferate most severely within one month after implantation of the stent, and hyperplasia of smooth muscle cells gradually slows down after one month of implantation, comparing to one month, the luminal stenosis rate changes little.

The zinc content in the zinc-containing portion is detected by a X-ray photoelectron spectroscopy (XPS) or a scanning electron microscopy (SEM), and includes the following steps: based on the actual position of the zinc-containing portion in the implantable device, a cross-section of the zinc-containing portion of the implantable device is exposed by one or more methods selected from the group consisting of resin embedding followed by grinding, directly grinding, and ion sputtering. Then the cross-section of the zinc-containing portion of the implantable device is observed by XPS or SEM. At least three sections of the zinc-containing portion of the implantable device are selected, a square region with the same area is selected in each section, and the zinc content in the square region is determined by an energy dispersive spectrometer- an accessory of XPS or SEM. And an average value is obtained by summarizing the zinc content in the square region of each section then averaging it as the zinc content of the zinc-containing portion. Methods of exposing the cross-section of a zinc-containing portion of an implantable device include, but are not limited to, the methods listed in embodiments in this disclosure. Methods of measuring the zinc content of a zinc-containing portion of an implantable device include, but are not limited to, the methods listed in embodiments in this disclosure.

An equiaxed structure is a polycrystalline structure having a grain shape of a polyhedron with a close dimension in each direction. For example, the equiaxed structure in an embodiment in the present disclosure refers to a polycrystalline structure having an average grain size ratio ranging from 1:1.2 to 1.2:1 in any two directions in any plane.

A non-equiaxed structure is a polycrystalline structure having a grain shape of a polyhedron with a different size in each direction. For example, the non-equiaxed structure in the present disclosure refers to a polycrystalline structure having an average grain size ratio less than 1:1.2 or more than 1.2:1 in at least two directions perpendicular to each other in at least one plane. Typical structures of a non-equiaxed structure include a deformation structure or a columnar structure, etc.

The ultrafine-grained structure in the present disclosure refers to a polycrystalline structure having an average grain size smaller than that of an equiaxed structure with a micro-grain size number of 14 in any direction in any plane. Further, when the average grain size of the ultrafine-grained structure in any direction in any plane is less than 100 nm, it is referred to as a nano-scale ultrafine-grained structure.

Whether a polycrystalline structure belongs to an equiaxed structure or a non-equiaxed structure can be determined by measuring an average grain size in certain directions in the polycrystalline structure based on GB/T 6394-2002 "Metal-methods for estimating the average grain size " or the ASTM E112-13 standard.

A method for measuring the average grain size of an equiaxed structure and determination of the micro-grain size number thereof is also carried out according to the afore mentioned standard. For example, an equiaxed structure with a micro-grain size number of 7-14 as determined by this standard has an average intercept in the range of 28.3 µm to 2.5 µm.

The average grain size of a non-equiaxed structure can also be measured according to the aforementioned standard. The average grain size of a non-equiaxed structure in any direction is greater than or equal to that of an equiaxed structure having a micro-grain size number of 14.

The sizes of a non-equiaxed structure and an equiaxed structure with a micro-grain size number of 7-14 can also be characterized by a metallographic microscope. Characterization is performed in at least two planes perpendicular to each other in space.

The average grain size of an ultrafine-grained structure can be characterized by SEM, a metallographic microscope or a transmission electron microscope (TEM)

An amorphous structure can be characterized by X-ray diffraction (XRD) or TEM.

In one embodiment, the thickness of the zinc-containing portion is greater than 100 nm. The thickness of the zinc-containing portion may affect the time period during which the zinc corrosion product may be maintained at an effective concentration to inhibit hyperplasia in the tissues surrounding the zinc-containing portion. When the thickness of the zinc-containing portion is greater than 100 nm, the zinc corrosion product may be maintained at an effective concentration in the tissues surrounding the zinc-containing portion for at least one month to inhibit hyperplasia of smooth muscle cells.

The thickness of the zinc-containing portion is determined by the following method: a part of a zinc-containing portion of an implantable device is embedded with resin and ground and polished on a metallographic specimen pre-mill. At least three cross sections perpendicular to the surface of the device are selected and observed by SEM. The position of the zinc-containing portion in the cross section is determined again by an energy dispersive spectrometer-an accessory of the SEM, and the thickness of the zinc-containing portion in at least three positions along the normal direction of the surface of the implantable device is detected. An average value is obtained by summarizing the thicknesses at multiple positions of the zinc-containing portion in each cross section then averaging it as the thickness of the zinc-containing portion.

The zinc is present in the zinc-containing portion in the form of elemental zinc.

The zinc-containing portion may be in direct contact with body fluid to ensure that the zinc-containing portion can be at least partially corroded after implantation. The substrate of the implantable device may be at least partially made of elemental zinc or a zinc alloy; the zinc-containing portion may also be a zinc-containing plating or coating that is at least partially applied to the surface of the substrate of the device.

The zinc-containing portion may also be in indirect contact with body fluid or may be in direct contact with body fluid after a period of time of implantation so as to ensure that the zinc-containing portion is at least partially corroded after implantation. The substrate of the implantable device may be made of polymers or other metals or alloys, and the substrate has a cavity in which the zinc-containing portion is disposed. Or another one or more degradable polymer coatings (e.g., polyester coatings, polyamino acid coatings, polyanhydride coatings, or co-polymerized and co-blended coatings or graft-modified coatings of other polymers), corrodible metal layers, soluble coatings, or other porous and permeable coatings may also be applied to the surface of a zinc-containing plating or coating on the surface of the substrate of the implantable device, such that the zinc-containing portion can be in indirect contact with body fluid through the pores of the layer or layers above, or can be in direct contact with body fluid after the layer is or layers are degraded and corroded.

According to the invention, the substrate includes the zinc-containing portions or is at least partially in contact with the zinc-containing portion.

In one embodiment, the substrate is in contact with the zinc-containing portion in a manner selected from at least one of the following: The zinc-containing portion at least partially covers the surface of the substrate; or the substrate is provided with a gap, a groove or a hole in which the zinc-containing portion is disposed, or the substrate is provided with an cavity in which the zinc-containing portion is filled.

In one embodiment, the substrate is at least partially made of iron or an iron alloy.

In one embodiment, the substrate is at least partially made of a polymer.

In one embodiment, the polymer is at least one of degradable polymers, non-degradable polymers, or copolymers formed by copolymerizing at least one monomer forming the degradable polymer and at least one monomer forming the non-degradable polymer. The degradable polymer is selected from the group consisting of polylactic acid, polyglycolic acid, polycaprolactone, polysuccinate, or poly (β-hydroxybutyrate). The non-degradable polymer is selected from the group consisting of polystyrene, polytetrafluoroethylene, polymethylmethacrylate, polycarbonate or polyethylene terephthalate.

The implantable device of the present invention further includes an outer layer in contact with the zinc-containing portion. The outer layer has a porous structure, or includes at least one of corrodible metals or alloys, and water-soluble polymers.

The outer layer may include at least one corrodible metal or alloy, and the mass fraction of zinc in the alloy is preferably less than or equal to 0.1%.

In one embodiment, the outer layer further includes at least one active agent. The active agent is at least one of cytostatic agents, corticosteroids, prostacyclins, antibiotics, cytostatic agents, immunosuppressive agents, anti-inflammatory agents, anti-inflammatory agents, anti-angiogenic agents, anti-stenotic agents, anti-thrombotic agents, anti-sensitizing agents, or anti-tumor agents.

In one embodiment, the implantable device is a degradable implantable device, a partially degradable implantable device, or a non-degradable implantable device.

In one embodiment, the implantable device includes a vascular stent, a biliary stent, an esophageal stent, a urethral stent, or a vena cava filter.

In one embodiment, the vena cava filter is at least partially made of a shape memory material. The shape memory material includes a nickel titanium alloy.

The implantable device of the present invention has at least the following beneficial effects compared to the prior art which is achieved by matching the zinc content in the zinc-containing portion with the microstructure of zinc.

After the implantable device of embodiments in the present disclosure is implanted into a mammalian body, the zinc-containing portion is corroded to form a zinc corrosion product. By controlling the concentration of the zinc corrosion product in the tissue surrounding the zinc-containing portion, hyperplasia of smooth muscle cells of the tissue surrounding the zinc-containing portion can be effectively inhibited, and ulceration or necrosis of the tissue surrounding the zinc-containing portion caused by the concentration of the zinc corrosion product accumulated in the tissue exceeds the cytotoxic concentration can be avoided after implantation

### Brief Description of the Drawings

Fig. 1 is a schematic view showing the structure of the vena cava filter of Embodiment 1;
Fig. 2 is a pathological section of the tissue surrounding the vascular stent of Embodiment 5 after the stent being implanted into a coronary artery vessel of a minipig for one month;
Fig. 3 is a pathological section of the tissue surrounding the vascular stent of Embodiment 6 after the sent being implanted into a coronary artery vessel of a minipig for three month;
Fig. 4 is a pathological section of the tissues surrounding the vascular stent of Embodiment 10 after the stent being implanted into a coronary artery vessel of a minipig for one month;
Fig. 5 is a pathological section of the tissues surrounding the vascular stent of Comparative Embodiment 1 after the stent being implanted into a coronary artery vessel of a minipig for one month;
Fig. 6 is a pathological section of the tissue surrounding the vascular stent of Comparative Example 3 after the stent being implanted into a coronary artery vessel of a minipig for one month.

### Detailed Description of the Embodiments

First, the measurement methods related to the present embodiments is described as follows:
Measurement of Zinc Content in Zinc-Containing Portion of Implantable device

The zinc content in the zinc-containing portion is determined by a X-ray photoelectron spectroscopy (XPS) or a scanning electron microscopy (SEM) as follows: based on the actual position of the zinc-containing portion in the implantable device, a cross section of the zinc-containing portion of the implanted device is exposed by one or more methods selecting from the group consisting of resin embedding followed by grinding, direct grinding and ion sputtering. Then the cross-section was observed through XPS or SEM by randomly selecting a square region within the cross-section and the zinc content of the square region was measured by an energy dispersive spectrometer- an accessory of XPS or SEM. The above steps are repeated and measured the zinc content of square regions with the same area in at least three sections of the zinc-containing portion. An average value is obtained by summarizing the zinc content of the square region of each section then averaging it as the zinc content of the zinc-containing portion.

XPS is ESCALAB 250Xi X-ray photoelectron spectrometer from Thermo Fisher, and SEM is JSM6510 Scanning Electron Microscope from Japan Electronics Co., Ltd., or the like.

Measurement of Microstructure and Grain Size of Zinc in Zinc-Containing Portion of Implantable Device

The grain size of zinc in the zinc-containing portion of the implantable device is measured according to GB/T 6394-2002 or ASTM E112-13 and will not be described further herein.

Thickness Measurement of Zinc-Containing Portion of Implantable Device

The thickness of the zinc-containing portion was measured by the following method: A part of the zinc-containing portion of the implantable device is embedded and secured by resin and ground and polished on a metallographic specimen pre-mill until a section of the zinc-containing portion of the implantable device was exposed. At least three sections perpendicular to the surface of the device were selected and then observed using SEM. And the position of the zinc-containing portion in the cross section was determined again by an energy dispersive spectrometer- an accessory of the SEM, to measure the thickness of at least three positions in the zinc-containing portion along the normal direction of the surface of the implantable device. And an average value is obtained by summarizing the thicknesses at multiple positions of the zinc-containing portion in each section then averaging it as the thickness of the zinc-containing portion.

SEM is JSM6510 Scanning Electron Microscope from Japan Electronics Co., Ltd., or the like.

### Measurement of Stenosis Rate in Tissues Surrounding the Zinc-Containing Portion

The stenosis rate in the tissues surrounding the zinc-containing portion was measured by animal implantation experiments. Taking a vascular stent as an example, the measurement includes the following steps. Several Vascular stents were implanted into blood vessels of several test animals respectively. The vascular stent and the vascular tissues surrounding the vascular stent were then removed at a predetermined observation time, such as 1 month, 3 months, and 6 months respectively. After a pathological section of the stent together with the vascular tissues, the section was observed using a DM2500 microscope from LEICA, Germany, and the lumen area of the section in which the zinc-containing portion of the device was located and the original lumen area were measured. Then, the luminal stenosis rate = (original lumen area-existing lumen area)/original lumen area x 100%.

The implantable device provided by embodiments in the present disclosure is further described below with reference to the figures and embodiments. It can be understood that the following embodiments are only exemplary embodiments of the disclosure and are non-limiting.

All of the embodiments described herein below are to be considered as reference-embodiments, as none of the embodiments describes an implantable device which further comprises an outer layer in contact with the zinc-containing portion, said outer layer having a porous structure or comprising a corrodible metal or alloy or water-soluble polymer as defined in independent claim 1. Additionally, some of the embodiments are indicated as "not according to the claims" as they further do not include a zinc-containing portion in the form of elemental zinc or the microstructure of zinc in the zinc-containing portion is not as defined in independent claim 1.

### Embodiment 1

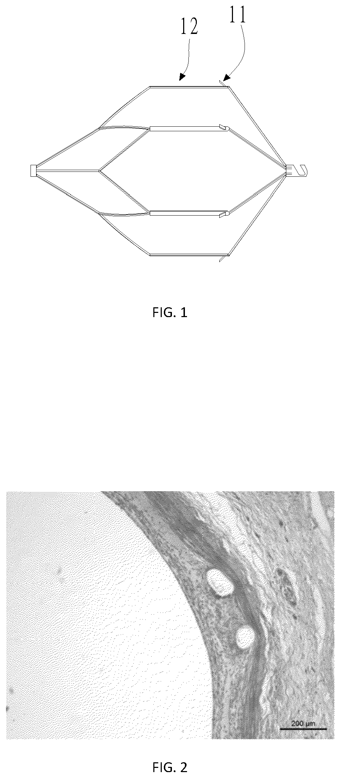

Figure 1 showed a vena cava filter made of a nitinol material. The filter was mainly composed of a number of supporting rods 11, one end of each support rod 11 being provided with a fixing anchor 12. After being implanted, the anchors 12 penetrated the inner wall of the blood vessel to fix the vena cava filter. After the fixing anchors 12 of the vena cava filter and the supporting rods 11 connected with the fixing anchor 12 being subjected to surface treatment, a zinc layer was plated by an electroplating method, thus the vena cava filter of this embodiment was obtained, the electroplating process parameters were as follows: composition of electroplating solution: zinc chloride 50 g/L, potassium chloride 150 g/L, boric acid 20 g/L, pH of the electroplating solution: 5, plating temperature: 20 °C, current density: 5 A/dm². In the vena cava filter of the embodiment, the zinc-containing portion was a zinc plating on the fixing anchor and the filter rod connected to it.

In the vena cava filter provided in Embodiment 1, the zinc content in the zinc-containing portion (i.e., the zinc plating on the fixing anchor and the filter rod connected to it) was 99.6 wt% as measured by the above measurement method, and the microstructure of zinc was a nano-scale ultrafine-grained structure. The thickness of the zinc-containing portion was 0.5 µm.

The vena cava filter provided in Embodiment 1 was implanted into the inferior vena cava of a dog. One month after implantation, the filter and the vascular tissues surrounding the filter were removed, the vascular tissues surrounding the anchors of the vena cava filter were separated, and a pathological analysis was conducted on the vascular tissues surrounding the anchors. The results showed that after the vena cava filter provided by Embodiment 1 being implanted into the animal for one month, there was no obvious hyperplasia of smooth muscle cells in the vascular tissues surrounding the anchors, and there was also no significant neointima attached to the anchors and the filter rods connected to it.

### Embodiment 2

The vascular stent of Embodiment 2 was made using a pure zinc material. In the vascular stent of the embodiment, the zinc-containing portion was the entire pure zinc stent.

The zinc content in the zinc-containing portion of the vascular stent provided in Embodiment 2 was 99.9 wt.% as measured by the above measurement method. The microstructure of zinc is an equiaxed structure with a micro-grain size number of 14. The thickness of the zinc-containing portion was 120 µm.

The vascular stent provided by Embodiment 2 was implanted into a coronary artery vessel of a minipig, maintaining an over-expansion ratio in the range of 1.1: 1 to 1.2: 1 during implantation. It was followed up one month after implantation, during which the stent and the vascular tissues surrounding the stent were removed and a pathological analysis was conducted on the vascular tissues. The pathological analysis results showed that the vascular stent provided by Embodiment 2 can effectively inhibit hyperplasia of smooth muscle cells of the vascular tissues surrounding the stent after the stent being implanted into an animal for one month, and endothelial cells grew normally, no cell necrosis occurred, and the luminal stenosis rate was 36% as measured by the above method.

### Embodiment 3

A zinc layer was uniformly plated on the surface of the 316 L stainless steel stent by an electroplating method. The electroplating process parameters were as follows to obtain the vascular stent of the embodiment: composition of electroplating solution: zinc chloride 50 g/L, potassium chloride 150 g/L, boric acid 20 g/L, pH of the electroplating solution: 5, electroplating temperature: 20 °C, current density: 5 A/dm². In the vascular stent of this embodiment, the zinc-containing portion was a zinc plating covering the entire surface of the 316 L stainless steel stent, and the thickness of the stainless steel stent was 120 mum.

The zinc content in the zinc-containing portion (i.e., the zinc plating) in the vascular stent provided in Embodiment 3 was 99 wt.% as measured by the above measurement method. The microstructure of zinc was an ultrafine-grained structure. The thickness of the zinc-containing portion was 1 µm.

The vascular stent of Embodiment 3 was implanted into a coronary artery vessel of a minipig, maintaining an over-expansion ratio in the range of 1.1: 1 to 1.2: 1 during implantation. It was then followed up 1 month after implantation. During the follow-up, the stent and the vascular tissues surrounding the stent were removed, and a pathological analysis of the tissues surrounding the stent showed that after the vascular stent provided by Embodiment 3 being implanted into the animal for one month, there was no obvious hyperplasia of smooth muscle cells in the tissues surrounding the vascular stent, and the endothelial cells of the vascular tissues grew normally, and also no tissue cell necrosis occurred around the stent struts. The vascular stenosis rate was 38% as measured by the above measurement method.

### Embodiment 4

A number of grooves were carved on the surface of the 316 L stainless steel vascular stent to a depth of 20 µm, and elemental zinc with the zinc content of 99 wt.% was tightly embedded into the grooves to obtain the vascular stent of the embodiment. In the vascular stent of the embodiment, the zinc-containing portion was elemental zinc embedded in the grooves, and the thickness of the stainless steel stent was 120 µm.

The zinc content in the zinc-containing portion (i.e., the zinc alloy embedded in the grooves) in the vascular stent provided in Embodiment 4 was 99 wt.% as measured by the above measurement method, the microstructure of zinc was a non-equiaxed crystal structure, and the thickness of the zinc-containing portion was 20 micrometers.

The vascular stent provided in Embodiment 4 was implanted into a coronary artery vessel of a minipig, maintaining an over-expansion ratio in the range of 1.1:1 to 1.2:1 during implantation. One month after implantation, the stent and the vascular tissue surrounding the stent were removed, and a pathological analysis was conducted on the vascular tissues. The results showed that after the vascular stent provided by the embodiment 4 being implanted into the animal for 1 month, there was no obvious hyperplasia of smooth muscle cells in the tissues surrounding the stent, and the endothelial cells of the vascular tissue grow normally, and also no tissue cell necrosis occurred around the stent struts. The vascular stenosis rate was 28% as measured by the above measurement method.

### Embodiment 5

A zinc layer was uniformly plated on the surface of a pure iron stent by an electroplating method. The electroplating process parameters were as follows to obtain the vascular stent of the embodiment: composition of electroplating solution: zinc chloride 50 g/L, potassium chloride 150 g/L, boric acid 20 g/L, pH of the electroplating solution: 5, electroplating temperature: 20 °C, current density: 5 A/dm². In the vascular stent of the embodiment, the zinc-containing portion was the zinc plating covering the entire surface of the pure iron stent, and the thickness of the pure iron stent was 60 µm.

The zinc content in the zinc-containing portion (i.e., the zinc plating) in the vascular stent provided by Embodiment 5 was 99 wt.% as measured by the above measurement method. The microstructure of zinc was an ultrafine-grained structure. The thickness of the zinc-containing portion was 1 µm.

The vascular stent of Embodiment 5 was implanted into a coronary artery vessel of a minipig, maintaining an over-expansion ratio in the range of 1.1:1 to 1.2:1 during implantation. It was then followed up 1 month after implantation. During the follow-up, the stent and the vascular tissues surrounding the stent were removed, a pathological analysis was conducted on the tissues surrounding the stent, and the pathological pictures were shown in figure 2. The pathological analysis results showed that after the vascular stent provided by Embodiment 5 being implanted into the animal for one month, there was no significant hyperplasia of smooth muscle cells in the tissues surrounding the stent, and endothelial cells of the vascular tissues grew normally, and also no tissue cell necrosis occurred around the stent struts. The vascular stenosis rate was 30% as measured by the above measurement method.

### Embodiment 6

A zinc layer was uniformly plated on the surface of the pure iron stent by an electroplating method. The electroplating process parameters were as follows to obtain the vascular stent of this embodiment: composition of electroplating solution: zinc oxide 15 g/L, ferrous sulfate·7H₂O 5 g/L, sodium hydroxide 100 g/L, triethanolamine 25 ml/L, temperature: 25 °C, current density: 1 A/dm². In the vascular stent of the embodiment, the zinc-containing portion was a zinc plating covering the entire surface of the pure iron stent, the thickness of the stent being 60 µm.

The zinc content in the zinc-containing portion (i.e., the zinc plating) in the vascular stent provided by Embodiment 6 was 80 wt.% as measured by the above measurement method, and the microstructure of zinc was an ultrafine-grained structure. The thickness of the zinc-containing portion was 1 µm.

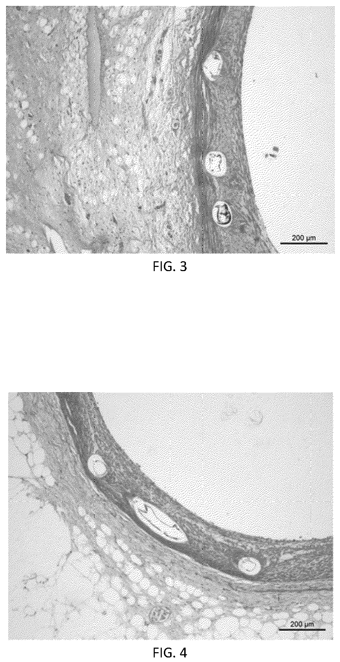

The vascular stent provided in Embodiment 6 was implanted into a coronary artery vessel of a minipig, maintaining an over-expansion ratio in the range of 1.1:1 to 1.2:1 during implantation. After 3 months of implantation, the stent and the vascular tissues surrounding the stent were removed and a pathological analysis was conducted on the vascular tissues, the pathological picture was shown in Fig. 3. The pathological analysis results showed that after the vascular stent provided by the embodiment 6 being implanted into the animal for 3 months, there was no significant hyperplasia of smooth muscle cells in the tissues surrounding the stent, and endothelial cells of the vascular tissues grew normally, and also no tissue cell necrosis occurred around the stent struts. The vascular stenosis rate was 30% as measured by the above measurement method.

### Embodiment 7

A zinc layer was uniformly plated on the surface of the pure iron stent by an electroplating method. The electroplating process parameters are as follows to obtain the vascular stent of the embodiment: composition of electroplating solution: zinc oxide 15 g/L, ferrous sulfate. 7H₂O 5 g/L, sodium hydroxide 100 g/L, triethanolamine 25 mL/L, temperature: 25 °C, current density: 1A/dm². In the vascular stent of the embodiment, the zinc-containing portion is a zinc plating covering the entire surface of the pure iron stent, the thickness of the pure iron stent being 60 µm.

The zinc content in the zinc-containing portion (i.e., the zinc plating) in the vascular stent provided in Embodiment 7 was 80 wt.% as measured by the above measurement method, and the microstructure of zinc was an ultrafine-grained structure. The thickness of the zinc-containing portion was 0.5 µm.

The vascular stent provided by Embodiment 7 was implanted into a coronary artery vessel of a minipig to maintain an over-expansion ratio in the range of 1.1:1 to 1.2:1 during implantation. After 3 months of implantation, the stent and the vascular tissues surrounding the stent were removed and a pathological analysis was conducted on the vascular tissues. The pathological analysis results showed that after the vascular stent provided by Embodiment 7 being implanted into the animal for three months, there was no significant hyperplasia of smooth muscle cells in the tissues surrounding the stent, and endothelial cells of the vascular tissues grew normally, and also no tissue cell necrosis occurred around the stent struts. The vascular stenosis rate was 38% as measured by the above measurement method.

### Embodiment 8 (not according to the claims)

A number of grooves were carved on the surface of the pure iron vascular stent to a depth of 20 µm, and zinc alloy with zinc content of 70 wt.% was tightly embedded into the grooves to obtain the vascular stent of the embodiment. In the vascular stent of the embodiment, the zinc-containing portion was zinc alloy embedded in the grooves, and the thickness of the stainless steel stent was 120 µm.

The zinc content in the zinc-containing portion (i.e., the zinc alloy embedded in the groove) in the vascular stent provided by Embodiment 8 was 70 wt.% as measured by the above measurement method, the microstructure of the zinc was an equiaxed crystal structure having a micro-grain size number of 10, and the thickness of the zinc-containing portion was 20 micrometers.

The vascular stent provided in Embodiment 8 was implanted into a coronary artery vascular of a minipig, maintaining an over-expansion ratio in the range of 1.1:1 to 1.2:1 during implantation. One month after implantation, the stent and the vascular tissue surrounding the stent were removed, and a pathological analysis was conducted on the vascular tissues. The results showed that after the stent provided by Embodiment 8 being implanted into the animal for one month, there was no significant hyperplasia of smooth muscle cells in the tissues surrounding the stent, and endothelial cells of the vascular tissues grew normally, and also no tissue cell necrosis occurred around the stent struts. The vascular stenosis rate was 19% as measured by the above measurement method.

### Embodiment 9 (not according to the claims)

The vascular stent of Embodiment 9 was made of a zinc alloy material. In the vascular stent of the embodiment, the zinc-containing portion was the entire zinc alloy stent.

The zinc content in the zinc-containing portion of the vascular stent provided by Embodiment 9 was 60 wt.% as measured by the above measurement method. The microstructure of zinc was an equiaxed structure with a micro-grain size number of 7. The thickness of the zinc-containing portion was 120 µm.

The vascular stent provided by Embodiment 9 was implanted into a coronary artery vessel of a minipig, maintaining an over-expansion ratio in the range of 1.1:1 to 1.2:1 during implantation. It was followed up one month after implantation, during which the stent and the vascular tissues surrounding the stent were removed, and a pathological analysis of the vascular tissues showed that after the stent provided by Embodiment 9 being implanted into the animal for one month, there was no significant hyperplasia of smooth muscle cells in the tissues surrounding the stent, and endothelial cells of the vascular tissues grew normally, and also no tissue cell necrosis occurred around the stent struts. The vascular stenosis rate was 35% as measured by the above measurement method.

### Embodiment 10 (not according to the claims)

The vascular stent of Embodiment 10 was made of a zinc alloy material. In the vascular stent of the embodiment, the zinc-containing portion was the entire zinc alloy stent.

The zinc content in the zinc-containing portion of the vascular stent provided by Embodiment 10 was 60 wt.% as measured by the above measurement method. The microstructure of zinc was an amorphous structure. The thickness of the zinc-containing portion was 120 µm.

The vascular stent provided by Embodiment 10 was implanted into a coronary artery vessel of a minipig, maintaining an over-expansion ratio in the range of 1.1:1 to 1.2:1 during implantation. It was followed up one month after implantation, during which the stent and the vascular tissue surrounding the stent were removed, and a pathological analysis was conducted on the vascular tissues, the results were shown in Figure 4. The pathological analysis results showed that after the stent provided by Embodiment 10 being implanted into the animal for one month, there was no significant hyperplasia of smooth muscle cells in the tissues surrounding the vascular stent, and endothelial cells of the vascular tissues grew normally, and also no tissue cell necrosis occurred around the stent struts. The vascular stenosis rate was 35% as measured by the above measurement method.

### Embodiment 11 (not according to the invention)

The vascular stent of Embodiment 11 was made of a zinc alloy material. In the vascular stent of the embodiment, the zinc-containing portion was the entire zinc alloy stent.

The zinc content in the zinc-containing portion of the vascular stent provided in Embodiment 11 was 50 wt.% as measured by the above test method. The microstructure of zinc was an amorphous structure. The thickness of the zinc-containing portion was 120 µm.

The vascular stent provided by Embodiment 11 was implanted into a coronary artery vessel of a minipig, maintaining an over-expansion ratio in the range of 1.1:1 to 1.2:1 during implantation. It was followed up one month of implantation, during which the stent and the vascular tissues surrounding the stent were removed, and a pathological analysis was conducted on the vascular tissues. The results showed that after the stent provided by Embodiment 11 being implanted into the animal for one month, there was no significant hyperplasia of smooth muscle cells in the tissues surrounding the vascular stent, and endothelial cells of the vascular tissues grew normally, and also no tissue cell necrosis occurred around the stent struts. The vascular stenosis rate was 30% as measured by the above test method.

### Embodiment 12 (not according to the claims)

The vascular stent of Embodiment 12 was made of a zinc alloy material. In the vascular stent of the embodiment, the zinc-containing portion was the entire zinc alloy stent.

The zinc content in the zinc-containing portion of the vascular stent provided by Embodiment 12 was 30 wt.% as measured by the above test method. The microstructure of zinc is an amorphous structure. The thickness of the zinc-containing portion was 120 µm.

The vascular stent provided by Embodiment 12 was implanted into a coronary artery vessel of a minipig, maintaining an over-expansion ratio in the range of 1.1:1 to 1.2:1 during implantation. It was followed up one month after implantation, during which the stent and the vascular tissues surrounding the stent were removed, and a pathological analysis was conducted on the vascular tissues. The results showed that after the stent provided by Embodiment 12 being implanted into the animal for one month, there was no significant hyperplasia of smooth muscle cells in the tissues surrounding the vascular stent, and endothelial cells of the vascular tissues grew normally, and also no tissue cell necrosis occurred around the stent struts. The vascular stenosis rate was 35% as measured by the above measurement method.

### Comparative Example 1

A number of grooves were carved on the surface of the 316 L stainless steel stent to a depth of 20 microns, and an elemental zinc with the zinc content of 99 wt.% was tightly embedded into the grooves to obtain the vascular stent of the comparative example, the thickness of the stainless steel stent was 120 microns. In the vascular stent of this comparative example, the zinc-containing portion was elemental zinc embedded into the grooves.

The zinc content in the zinc-containing portion (i.e., the zinc alloy embedded to in the grooves) in the vascular stent provided by Comparative Example 1 was 99 wt.% as measured by the above measurement method, the microstructure of zinc was amorphous and the thickness of the zinc-containing portion was 20 µm.

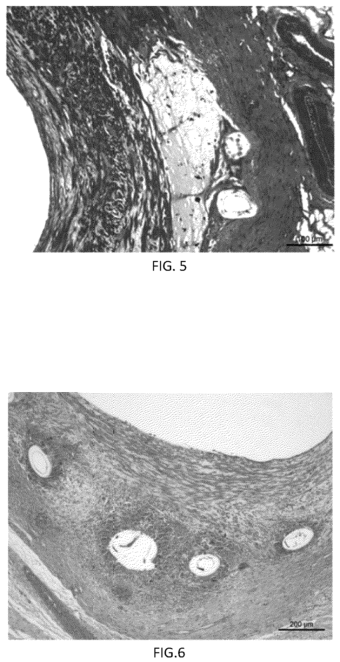

The vascular stent of Comparative Example 1 was implanted into a coronary artery vessel of a minipig. One month after implantation, the stent and the vascular tissue surrounding the stent were removed, and a pathological analysis was conducted on the vascular tissues, and the pathological pictures were shown in Fig. 5. As can be seen from FIG. 5, after the stent of Comparative Example 1 being implanted into the animal for one month, there was significant cell necrosis in the vascular tissues surrounding the vascular stent.

By comparing the two pathological analysis results of the vascular tissues surrounding the vascular stent respectively provided by Embodiment 4 and Comparative Example 1 one month after the stents being respectively implanted into an animal, it was found that in the vascular stent of Comparative Example 1, since the zinc content in the zinc-containing portion was not matched with the microstructure of zinc, the peak concentration of zinc corrosion products accumulated in the surrounding tissues exceeded the concentration that is toxic to normal tissue cells, and a significant cell necrosis occurred in the surrounding tissues. While in the vascular stent provided by Embodiment 4, by matching the zinc content in the zinc-containing portion on the surface of the stent with the microstructure of zinc, the mass of zinc corrosion products generated per unit time can be adjusted, such that the generated zinc corrosion products can inhibit hyperplasia of smooth muscle cells of vascular tissues surrounding the stent without causing cell necrosis.

### Comparative Example 2

Zinc was uniformly plated on the surface of the 316 L stainless steel stent by an electroplating process to obtain the vascular stent of Comparative Example 2, the electroplating process parameters were as follows: composition of electroplating solution: zinc chloride 50 g/L, potassium chloride 150 g/L, boric acid 20 g/L, pH of the electroplating solution: 5, electroplating temperature: 20 °C, current density: 5 A/dm². In the vascular stent of Comparative Example 2, the zinc-containing portion was a zinc plating covering the entire surface of the pure iron stent, the stainless steel stent having a thickness of 120 µm.

The content of zinc in the zinc-containing portion (i.e., the zinc plating) in the vascular stent of Comparative Example 2 was 99 wt.% as measured by the above measurement method, and the microstructure of the zinc was an ultrafine-grained structure. The thickness of the zinc-containing portion was 80 nm.

The vascular stents of Comparative Example 2 were implanted into a coronary artery vascular of a minipig, maintaining an over-expansion ratio in the range of 1.1:1 to 1.2:1 during implantation. The stents and the vascular tissues surrounding the stent were removed at 14 days and 1 month after implantation, respectively, and the vascular tissues were analyzed pathologically. The results of the pathological analysis showed that the smooth muscle cells of the tissues surrounding the stent were effectively inhibited at 14 days after implantation without cell necrosis, but hyperplasia of smooth muscle cells of the tissue surrounding the stent occurred obviously at 1 month after implantation, and the vascular stenosis rate was 44% as measured by the above method. Endothelial cells grew normally without cell necrosis.

The pathological analysis results of Comparative Example 2 showed that, in the vascular stent of Comparative Example 2, hyperplasia of smooth muscle cells in the tissues surrounding the zinc-containing portion can be inhibited by matching the zinc content in the zinc-containing portion with the microstructure of zinc, but the effect of inhibiting hyperplasia cannot be continuously maintained within one month due to the small thickness of the zinc-containing portion.

### Comparative Example 3

The vascular stent of Comparative Example 3 was made of a zinc alloy material. In the vascular stent of this comparative example, the zinc-containing portion was the entire zinc alloy stent.

The zinc content in the zinc-containing portion of the vascular stent provided by Comparative Example 3 was 30 wt.% as measured by the above measurement method. The microstructure of zinc is an equiaxed structure with a micro-grain size number of 10. The thickness of the zinc-containing portion was 120 µm.

The vascular stent of Comparative Example 3 was implanted into a coronary artery vessel of a minipig, maintaining an over-expansion ratio in the range of 1.1:1 to 1.2:1 during implantation. One month after implantation, the stent and its vascular tissue surrounding the stent were removed and a pathological analysis was conducted on the vascular tissues, and the results were shown in Fig. 6. As can be seen from FIG. 6, when the vascular stent of Comparative Example 3 was implanted into an animal for 1 month, smooth muscle cells of vascular tissues surrounding the stent were proliferated seriously, and the stenosis rate of blood vessels was 43% as measured by the above method. Endothelial cells grew normally without cell necrosis.

By comparing the two pathological analysis results of the vascular tissues around the vascular stent respectively provided by Embodiment 12 and Comparative Example 3 one month after the stent being respectively implanted into an animal, it was found that in the vascular stent of Comparative Example 3, since the zinc content in the zinc-containing portion was not matched with the microstructure of zinc, the zinc corrosion product concentration in the surrounding tissues was too low to inhibit hyperplasia of smooth muscle cells in the tissues surrounding the zinc-containing portion. While in the vascular stent provided by Embodiment 12, by matching the zinc content in the zinc-containing portion on the surface of the stent with the microstructure of zinc, the mass of zinc corrosion products generated per unit time can be adjusted, such that the generated zinc corrosion product can inhibit hyperplasia of smooth muscle cells of vascular tissues surrounding the stent without causing cell necrosis.

### Comparative Example 4

The stent used in this comparative example was a 316 L stainless steel stent having a thickness of 120 µm.

The vascular stent of Comparative Example 4 was implanted into a coronary artery vessel of a minipig, maintaining an over-expansion in the range of 1.1:1 to 1.2:1 during implantation. One month after implantation, the stent and the vascular tissue surrounding the stent were removed, and a pathological analysis of the vascular tissue showed that after the vascular stent of Comparative Example 4 being implanted for one month, the smooth muscle cells in the vascular tissues surrounding the stent proliferated severely, and the vascular stenosis rate was 45% as measured by the above method. Endothelial cells grew normally without cell necrosis.

As can be seen from Comparative Example 4, when there is no zinc corrosion product to inhibit hyperplasia of smooth muscle cells surrounding the stent struts, hyperplasia of smooth muscle cells occurred seriously, resulting in a high rate of vascular stenosis.

In summary, by matching the zinc content in the zinc-containing portion of the implanted device with the microstructure of zinc, the mass of the zinc corrosion product generated per unit time can be controlled, such that after the implantable device of the disclosure being implanted into an animal body, the concentration of the zinc corrosion product in the tissues surrounding the zinc-containing portion can inhibit hyperplasia of smooth muscle cells without causing death of smooth muscle cells, endothelial cells or normal tissue cells, so as to prevent tissue necrosis. However, it should be noted that if the local environment where the zinc-containing portion of the implantable device exists is a low pH environment, the low pH environment results from factors other than the mass and the microstructure of the zinc-containing portion of the apparatus, i.e., in addition to the zinc-containing portion of the device, results from degradation, dissolution of the device itself and/or other substances carried by the device, or chemical reaction thereof with other substance in vivo, for example, a low pH environment was caused by degradation of a degradable polylactic acid coating carried by a vascular stent. In this environment, the corrosion rate of the zinc-containing portion of the implantable device will be faster. Although the low pH value environment can accelerate the corrosion of the zinc-containing portion, if the corrosion rate of the zinc-containing portion is still controlled by the own features (ingredients and microstructure) of the zinc-containing portion, the own features of the zinc-containing portion of the corresponding implantable device should be adjusted towards the lower limit of the corrosion rate; if the corrosion rate of the zinc-containing portion has been controlled by a low pH environment, this disclosure is no longer applicable to this situation.

In addition, when the zinc content in the zinc-containing portion of the implantable device is in the range of [80,100] wt.%, and the microstructure of zinc in the zinc-containing portion is ultrafine-grained, the luminal stenosis rate after implantation of the device can be further reduced.

In addition, in embodiments in the disclosure, the time for maintaining the effective concentration of zinc corrosion products in the surrounding tissues is controlled by controlling the thickness of the zinc-containing portion (a thickness of greater than 100 nm), so that hyperplasia of smooth muscle cells in the tissues surrounding the zinc-containing portion can be effectively inhibited within one month after the implantable device being implanted into an animal.

It can be understood that, embodiments in the present disclosure are schematically illustrated by the implantable devices provided by Embodiments 1 to 8 respectively, each zinc-containing portion of which is formed of elemental zinc or zinc alloy with the same composition and same ingredient (the zinc alloy is an alloy formed by zinc and at least one of iron, magnesium, manganese, copper or strontium, or an alloy formed by zinc doped with at least one of carbon, nitrogen, oxygen, boron or silicon), In embodiments provided by the disclosure, the implantable device can also have a number of zinc-containing portions, each zinc-containing portion having a different composition. For example, in a number of zinc-containing portions of an implantable device, part of zinc-containing portions may be formed of elemental zinc, and the remaining of zinc-containing portions may be comprised of a zinc alloy. As another example, in the number of zinc-containing portions of the implantable device, the zinc content and the microstructure of zinc in each zinc-containing portion may also be different from each other. As another example, by controlling the plating process conditions, the zinc microstructure may have a variety of different structural morphology in a certain zinc-containing portion of the implantable device. As long as the zinc content in each zinc-containing portion is matched with the microstructure of zinc, the zinc concentration in the tissue surrounding the zinc-containing portion can be controlled, so that the purpose of inhibiting hyperplasia of smooth muscle cells in the surrounding tissues can be achieved.

It can also be understood that the embodiments of the present disclosure are only schematically illustrated by the implantable devices provided by Embodiment 1 to 8 respectively, in which elemental zinc or zinc alloy was used as a plating, or the entire device substrate was formed of elemental zinc or zinc alloy. The implanted device includes a substrate, and the substrate at least partially includes the zinc-containing portion or at least partially is in contact with the zinc-containing portion, and in the technical solution provided by the disclosure, the zinc-containing portion (i.e. the zinc plating) can only partially cover the surface of the substrate; or the substrate is provided with a gap, a groove or a hole in which the zinc-containing portion (for example, elemental zinc) is disposed; or the substrate is provided with an cavity in which the zinc-containing portion is filled. In the above embodiment, as long as the zinc content in the zinc-containing portion is matched with the microstructure of zinc, hyperplasia of smooth muscle cells of the tissues surrounding the zinc-containing portion can be inhibited without causing necrosis of normal tissue cells.

It can also be understood that the substrate may be at least partially made of iron or an iron alloy, the substrate is at least partially made of a polymer selected from at least one of degradable polymers, non-degradable polymers or copolymers formed by copolymerizing of at least one monomer forming the degradable polymer and at least one monomer forming the non-degradable polymer, the degradable polymer is selected from the group consisting of polylactic acid, polyglycolic acid, polycaprolactone, polysuccinate or poly (β-hydroxybutyrate), the non-degradable polymer is selected from the group consisting of polystyrene, polytetrafluoroethylene, polymethylmethacrylate, polycarbonate or polyethylene terephthalate. It should also be understood that the implantable device of the present invention further includes an outer layer in contact with the zinc-containing portion, the outer layer has a porous structure, or the outer layer includes at least one of corrodible metals or alloys, or water-soluble polymers.

For example, the outer layer may further includes at least one corrodible metal or alloy, and the mass fraction of zinc thereof is less than or equal to 0.1%. In another example, the outer layer may include at least partially water-soluble polymers, or the outer layer may have a porous structure so long as the outer layer can ensure that the zinc-containing portion can be in direct or indirect contact with body fluid.

It can also be understood that the embodiments provided by the present disclosure may also be applicable to other implantable devices such as tracheal stents, biliary stents, esophageal stents, urethral stents or vena cava filters.

## Claims

1. An implantable device comprising at least one corrodible zinc-containing portion, wherein the zinc content in the at least one zinc-containing portion ranges from (70,100] wt.%, and
the microstructure of zinc in the zinc-containing portion is at least one of an non-equiaxed structure, an ultrafine-grained structure or an equiaxed structure having a micro-grain size number of 7-14,
wherein the implantable device comprises a substrate, the substrate including the zinc-containing portion or at least partially being in contact with the zinc-containing portion, and
wherein the implantable device further comprises an outer layer in contact with the zinc-containing portion, wherein the outer layer has a porous structure, or the outer layer comprises at least one of corrodible metals or alloys or water-soluble polymers, and
wherein zinc is present in the zinc-containing portion in the form of elemental zinc.

2. An implantable device according to claim 1, wherein the substrate is at least partially made of iron or an iron alloy or is made of a polymer.

3. The implantable device of claim 1, wherein the zinc-containing portion has a thickness greater than 100 nm.

4. The implantable device of claim 1, wherein the substrate is in contact with the zinc-containing portion in a manner selected from at least one of the following: the zinc-containing portion at least partially covers the surface of the substrate; or the substrate is provided with a gap, a groove or a hole in which the zinc-containing portion is disposed; or the substrate being provided with an cavity in which the zinc-containing portion is filled.

5. The implantable device of claim 2, wherein the polymer from which the substrate is at least partially made is selected from at least one of a degradable polymer, a non-degradable polymer, or a copolymer formed by copolymerizing at least one monomer forming the degradable polymer and at least one monomer forming the non-degradable polymer, the degradable polymer is selected from the group consisting of polylactic acid, polyglycolic acid, polycaprolactone, polycarbonate, polysuccinate or poly (β-hydroxybutyrate), the non-degradable polymer is selected from the group consisting of polystyrene, polytetrafluoroethylene, polymethylmethacrylate or polyethylene terephthalate.

6. The implantable device of claim 1, wherein the outer layer comprises at least one corrodible metal or alloy, and the mass fraction of zinc in the metal or alloy is less than or equal to 0.1%.

7. The implantable device of claim 1, wherein the outer layer further comprises at least one active agent selected from at least one of cytostatic agents, corticosteroids, prostacyclins, antibiotics, cytostatic agents, immunosuppressive agents, anti-inflammatory agents, anti-angiogenic agents, anti-stenosis agents, anti-thrombotic agents, anti-sensitizing agents, or anti-tumor agents.

8. The implantable device of claim 1, wherein the implantable device is a degradable implantable device, a partially degradable implantable device, or a non-degradable implantable device.

9. The implantable device of claim 1, wherein the implantable device comprises a vascular stent, a biliary stent, an esophageal stent, a urethral stent, or a vena cava filter, in particular wherein the vena cava filter is at least partially made of a shape memory material, the shape memory material comprising a nickel titanium alloy.

## Patentansprüche

1. Implantierbare Vorrichtung, die mindestens einen korrodierbaren zinkhaltigen Teil umfasst, wobei der Zinkgehalt in dem mindestens einen zinkhaltigen Teil im Bereich von (70,100] Gew.-% liegt und
die Mikrostruktur von Zink in dem zinkhaltigen Teil eine nicht äquiaxiale Struktur und/oder eine ultrafeinkörnige Struktur und/oder eine äquiaxiale Struktur mit einer Mikrokorngrößenzahl von 7-14 ist,
wobei die implantierbare Vorrichtung ein Substrat umfasst, wobei das Substrat den zinkhaltigen Teil aufweist oder sich zumindest teilweise in Kontakt mit dem zinkhaltigen Teil befindet, und
wobei die implantierbare Vorrichtung ferner eine Außenschicht umfasst, die sich in Kontakt mit dem zinkhaltigen Teil befindet, wobei die Außenschicht eine poröse Struktur aufweist oder die Außenschicht korrodierbare Metalle und/oder Legierungen und/oder wasserlösliche Polymere umfasst, und
wobei Zink in dem zinkhaltigen Teil in Form von elementarem Zink vorliegt.

2. Implantierbare Vorrichtung nach Anspruch 1, bei der das Substrat zumindest teilweise aus Eisen oder einer Eisenlegierung oder aus einem Polymer besteht.

3. Implantierbare Vorrichtung nach Anspruch 1, bei der der zinkhaltige Teil eine Dicke von mehr als 100 nm aufweist.

4. Implantierbare Vorrichtung nach Anspruch 1, bei der sich das Substrat in einer Weise mit dem zinkhaltigen Teil in Kontakt befindet, die aus mindestens einer der folgenden ausgewählt ist: der zinkhaltige Teil bedeckt zumindest teilweise die Oberfläche des Substrats; oder das Substrat ist mit einem Spalt, einer Rille oder einem Loch versehen, in dem bzw. der der zinkhaltige Teil angeordnet ist; oder das Substrat ist mit einem Hohlraum versehen, in den der zinkhaltige Teil gefüllt ist.

5. Implantierbare Vorrichtung nach Anspruch 2, bei der das Polymer, aus dem das Substrat zumindest teilweise besteht, aus einem abbaubaren Polymer und/oder einem nicht abbaubaren Polymer und/oder einem Copolymer ausgewählt ist, das durch Copolymerisieren mindestens eines Monomers, das das abbaubare Polymer bildet, und mindestens eines Monomers gebildet ist, das das nicht abbaubare Polymer bildet, das abbaubare Polymer aus der aus Polymilchsäure, Polyglykolsäure, Polycaprolacton, Polycarbonat, Polysuccinat oder Poly(β-hydroxybutyrat) bestehenden Gruppe ausgewählt ist, das nicht abbaubare Polymer aus der aus Polystyrol, Polytetrafluorethylen, Polymethylmethacrylat oder Polyethylenterephthalat bestehenden Gruppe ausgewählt ist.

6. Implantierbare Vorrichtung nach Anspruch 1, bei der die Außenschicht mindestens ein korrodierbares Metall oder eine korrodierbare Legierung umfasst und der Massenanteil von Zink im Metall oder in der Legierung kleiner oder gleich 0,1% ist.

7. Implantierbare Vorrichtung nach Anspruch 1, bei der die Außenschicht ferner mindestens einen Wirkstoff umfasst, der aus mindestens einem der folgenden ausgewählt ist: Zytostatika, Corticosteroide, Prostacycline, Antibiotika, Zytostatika, Immunsuppressiva, entzündungshemmende Mittel, antiangiogene Mittel, Mittel gegen Stenose, Antithrombotika, Antisensibilisierungsmittel oder Tumorhemmer.

8. Implantierbare Vorrichtung nach Anspruch 1, wobei die implantierbare Vorrichtung eine abbaubare implantierbare Vorrichtung, eine teilweise abbaubare implantierbare Vorrichtung oder eine nicht abbaubare implantierbare Vorrichtung ist.

9. Implantierbare Vorrichtung nach Anspruch 1, wobei die implantierbare Vorrichtung einen Gefäßstent, einen Gallenstent, einen Speiseröhrenstent, einen Harnröhrenstent oder einen Vena-Cava-Filter umfasst, insbesondere wobei der Vena-Cava-Filter zumindest teilweise aus einem Formgedächtnismaterial besteht, wobei das Formgedächtnismaterial eine Nickel-Titan-Legierung umfasst.

## Revendications

1. Dispositif implantable comprenant au moins une partie corrosive contenant du zinc, la teneur en zinc dans ladite au moins une partie contenant du zinc étant comprise entre (70, 100] % en poids, et
la microstructure de zinc dans la partie contenant du zinc étant une structure non équiaxe et/ou une structure à grains ultrafins et/ou une structure équiaxe présentant un indice de grosseur de micro-grain de 7 à 14,
le dispositif implantable comprenant un substrat, le substrat présentant la partie contenant du zinc ou étant au moins partiellement en contact avec la partie contenant du zinc, et
le dispositif implantable comprenant en outre une couche extérieure en contact avec la partie contenant du zinc, la couche extérieure présentant une structure poreuse, ou la couche extérieure comprenant des métaux corrosifs et/ou des alliages et/ou des polymères solubles dans l'eau, et
le zinc étant présent dans la partie contenant du zinc sous forme de zinc élémentaire.

2. Dispositif implantable selon la revendication 1, dans lequel le substrat est au moins partiellement réalisé en fer ou en un alliage de fer ou est réalisé en un polymère.

3. Dispositif implantable selon la revendication 1, dans lequel la partie contenant du zinc présente une épaisseur supérieure à 100 nm.

4. Dispositif implantable selon la revendication 1, dans lequel le substrat est en contact avec la partie contenant du zinc d'une manière choisie parmi au moins l'une des suivantes : la partie contenant du zinc recouvre au moins partiellement la surface du substrat, ou le substrat est pourvu d'une fente, d'une rainure ou d'un trou dans laquelle/lequel la partie contenant du zinc est agencée, ou le substrat est pourvu d'une cavité dans laquelle la partie contenant du zinc est remplie.

5. Dispositif implantable selon la revendication 2, dans lequel le polymère à partir duquel le substrat est au moins partiellement réalisé est choisi parmi un polymère dégradable et/ou un polymère non-dégradable et/ou un copolymère réalisé par copolymérisation d'au moins un monomère formant le polymère dégradable et d'au moins un monomère formant le polymère non-dégradable, le polymère dégradable étant choisi dans le groupe constitué d'acide polylactique, d'acide polygycolique, de polycaprolactone, de polycarbonate, de polysuccinate ou de poly (β-hydroxybutyrate), le polymère non-dégradable étant choisi dans le groupe constitué de polystyrène, de polytétrafluoroéthylène, de polyméthacrylate de méthyle ou de polytéréphtalate d'éthylène.

6. Dispositif implantable selon la revendication 1, dans lequel la couche extérieure comprend au moins un métal ou un alliage corrosif et la fraction massique de zinc dans le métal ou l'alliage est inférieure ou égale à 0,1%.

7. Dispositif implantable selon la revendication 1, dans lequel la couche extérieure comprend en outre au moins un agent actif choisi parmi les agents cytostatiques et/ou les corticostéroïdes et/ou les prostacyclines et/ou les antibiotiques et/ou les agents cytostatiques et/ou les agents immunosuppresseurs et/ou les agents anti-inflammatoires et/ou les agents anti-angiogéniques et/ou les agents anti-sténose et/ou les agents anti-thrombotiques et/ou les agents anti-sensibilisants et/ou les agents anti-tumoraux.

8. Dispositif implantable selon la revendication 1, dans lequel le dispositif implantable est un dispositif implantable dégradable, un dispositif implantable partiellement dégradable ou un dispositif implantable non-dégradable.

9. Dispositif implantable selon la revendication 1, le dispositif implantable comprenant un stent vasculaire, un stent biliaire, un stent oesophagien, un stent urétéral ou un filtre de veine cave, le filtre de veine cave étant en particulier au moins partiellement réalisé en un matériau à mémoire de forme, le matériau à mémoire de forme comprenant un alliage de nickel et de titane.
